# EUROPEAN PATENT SPECIFICATION

(11) **EP 4 175 616 B1**
(45) Date of publication and mention of the grant of the patent: **25.03.2026**
(21) Application number: 21748658.8
(22) Date of filing: 01.07.2021
(51) Int. Cl.: A61K 9/00, A61K 47/02, A61K 33/00, A61K 33/18, A61M 3/02, A61M 11/00, A61M 15/08, A61F 7/00, A61H 35/04

(54) **USE OF SALT-BROMINE-IODINE WATERS FOR THE NASAL TREATMENT OF THE INFLAMMATION OF THE THREE AREAS OF THE UPPER AIRWAYS AND MEDICAL DEVICES ADAPTED TO SUCH A TREATMENT**
VERWENDUNG VON SALZBROMJODWASSER ZUR NASALEN BEHANDLUNG DER ENTZÜNDUNG DER DREI BEREICHE DER OBEREN ATEMWEGE UND MEDIZINISCHE GERÄTE DAFÜR
UTILISATION D'EAUX DE SEL-BROME-IODE POUR LE TRAITEMENT NASAL DE L'INFLAMMATION DES TROIS ZONES DES VOIES RESPIRATOIRES SUPÉRIEURES ET DISPOSITIFS MÉDICAUX ADAPTÉS À UN TEL TRAITEMENT

(30) Priority: 01.07.2020 IT 202000015844
(43) Date of publication of application: 10.05.2023
(73) Proprietor: ADL FARMACEUTICI S.R.L., 20129 Milano (MI) (IT)
(72) Inventor: DE LAURENTIIS, Antonio, 80123 Napoli (NA) (IT)
(74) Representative: Sarpi, Maurizio
(86) International application number: PCT/IB2021/055907
(87) International publication number: WO 2022/003618

(56) References cited:
- BARBIERI M ET AL: "[Behavior of serum IgE and IgA in patients with allergic rhinitis treated with iodine bromide thermal water]", ACTA OTORHINOLARYNGOLOGICA ITALICA, PACINI EDITORE, PISA, IT, vol. 22, no. 4, August 2002 (2002-08-01), pages 215 - 219, XP009192956, ISSN: 0392-100X
- PASSALI F M ET AL: "Effetti terapeutici dell'acqua termale salsobromoiodica di Salsamoggiore nel trattamento delle flogosi naso-sinusali ricorrenti e croniche [Efficacy of inhalation therapy with water of Salsomaggiore (Italy) in chronic and recurrent nasosinusal inflammation treatment]", LA CLINICA TERAPEUTICA, ITALY, vol. 159, no. 3, May 2008 (2008-05-01), pages 175 - 180, XP009192960, ISSN: 1972-6007
- CULIG JOSIP ET AL: "Efficiency of hypertonic and isotonic seawater solutions in chronic rhinosinusitis", MEDICINSKI GLASNIK, vol. 7, no. 2, August 2010 (2010-08-01), Bosnia and Herzegovina, pages 116 - 123, XP055785999, ISSN: 1840-0132
- SARAH KELLER ET AL: "Thermal Water Applications in the Treatment of Upper Respiratory Tract Diseases: A Systematic Review and Meta-Analysis", JOURNAL OF ALLERGY, vol. 2014, June 2014 (2014-06-01), pages 1 - 17, XP055409293, ISSN: 1687-9783, DOI: 10.1155/2014/943824
- ANONYMOUS T. R.: "LMA MAD Nasal(TM) - Intranasal Mucosal Atomization Device", TELEFLEX, 2013, pages 1 - 2, XP055786246, Retrieved from the Internet <URL:https://www.capesmedical.co.nz/media/mad300.pdf> [retrieved on 20210316], DOI: 10.1542/peds.2010-0616

## Description

The present invention relates to the management of pathological conditions linked to inflammatory, allergic and infectious events of the upper airways, which can mainly affect children and the elderly. In particular, the object of the invention relates to the combined use of isotonic and hypertonic thermal waters for both the irrigation and medication of the rhino-sinus-pharyngeal area, affecting three fundamental pathogenetic aspects: blocking, inflammation, and infection.

According to the invention, irrigating the nose with an isotonic solution and, subsequently, medicating it with a hypertonic solution, the unblocking, anti-inflammation and disinfection of the nasal area is obtained, on the health of which the "well-being" of the entire respiratory system depends.

The combined use of two different medical devices which allow performing the two different operating steps, that of nasal irrigation, indicated for daily hygiene, and that for the nebulization of medicines in solution, both of the type comprising a syringe connected to a nasal nozzle, is also an object of the invention. In the first case, a dispensing nozzle with several outlet holes is used so that the solution is capable of reaching the walls of both nasal cavities, in the second case a nebulizer nozzle capable of producing particles of a size between 70 and 10 microns, with an MMD preferably equal to 68.9 microns ± 6.7 microns, where MMD, the average diameter in volume, measured in terms of volume (or mass), indicates the value for which 50% of the total volume of the sprayed liquid is characterized by drops of smaller diameters than the average diameter and the remaining 50% by larger diameters.

The size of these particles are the most suitable for medication, as they affect only the three physio-pathological sites and minimize the risk that the aerosol reaches the lower respiratory tract and alveoli.

### SCOPE OF THE INVENTION

The inflammation, infection and allergy of the upper airways are the most relevant and most involved physio-pathological mechanisms in respiratory diseases, especially in childhood and senescence. The clinical features of diseases of the upper airways vary significantly according to the geographic area studied (1,2).

### Functional anatomy and physio-pathology

The airways should be considered overall as a functional unit, as there is strong evidence that the upper airways and the lower airways are closely connected to each other (5,6). The upper and lower airways share common epidemiological, anatomical, physiological and physio-pathological mechanisms. A paradigmatic example is a rhino-bronchial syndrome characterized by a spread of inflammatory and infectious events from the nose to the bronchi (7,8). In the rhino-sinus-pharyngeal area there are three important physio-pathological sites: i) the ostiomeatal complex, ii) the sphenoethmoidal recess and iii) the nasopharynx. Located on the lateral wall of the nose, the ostiomeatal complex is the space where the anterior rhino-sinus system conducts the drainage of the secretions thereof. Located behind the ostiomeatal complex, the phenoethmoidal recess drains the posterior rhino-sinus system. The nasopharynx is the site where the first physio-pathological step (such as posterior rhinorrhea) of descending laryngeal-tracheal-bronchial diseases begins. The adenoids are also found in the nasopharynx. Inflammation / infection of the nasopharynx is also implicated in the pathogenesis of otitis media. Furthermore, the nasopharynx is the site of the so-called "microbiological bank" as well as being the place where bacterial biofilms are formed (6). Correct ventilation and effective mucociliary clearance of these three physio-pathological sites guarantee the *"health"* of the entire respiratory system. In these sites, the filter of the inhaled air occurs, as well as the enrichment with rhino-sinus nitric oxide, which in turn regulates ciliary motility, which can prevent viral replication and regulate bronchial muscle tone (11,12). Therefore, the obstruction of the ostiomeatal complex, sphenoethmoidal recess and nasopharynx is the first essential pathogenetic step in the cascade of inflammatory events of the rhino-sinus-pharyngeal area, often complicated by infections.

The infections can involve the ostiomeatal complex and/or the sphenoethmoidal recess, causing anterior and/or posterior rhino-sinusitis (10), causing posterior rhinorrhea, which involves the nasopharynx microbial bank, which, in turn, can spread to the rhino-tympanic tube unit, causing otitis media (13), or to the middle-lower airways, with post-nasal drip, in which the secretions (containing microbes and proinflammatory substances) can spread to the middle-lower airways, causing rhino-bronchial syndrome (14).

The inflammation of the respiratory mucosa is the main pathogenic factor which induces the blockage of the airways (15). Infectious diseases mainly affect younger children, due to the immunological immaturity thereof, and the elderly, due to age-related immunodeficiency.

In contrast, inflammatory diseases and allergies primarily affect older children, adolescents and adults (10,14). Acute infectious diseases essentially have a viral origin, while the recurrent and chronic forms have mainly a bacterial origin: this is due to the lack of recovery of the acute infection, supported by local causes (hypertrophy of the adenoids, anatomical and functional malformations) and/or systemic causes (allergy, mucociliary changes, immunological defect) (14,15). Biofilm is an important local cause of recurrent disease: biofilm is a strategic mode of survival for bacteria and is the main cause of the resistance thereof to systemic antibiotic therapy (16).

A notable presence of biofilms has been demonstrated in the nasopharynx of children suffering from recurrent respiratory infections of the upper and lower airways (17). The biofilm can release, from time to time, numerous bacterial colonies which cause the recurrence of respiratory infections. Both infectious and inflammatory diseases share the same physio-pathological mechanisms: the respiratory mucosa shows a wide submucosal diffusion of immune-inflammatory cells which amplify inflammation (15,18). In this context, allergic inflammation can promote a vicious cycle which allows the predisposition to infection relapse and increases the severity of symptoms, such as:
- congestion and nasal blockage;
- inspiratory dyspnea;
- anterior and posterior rhinorrhea;
- sneezing and itching;
- cough,
- hyposmia and conjunctival disorders.

Among these symptoms, nasal congestion, with consequent blockage and inspiratory dyspnea, are very troublesome.

It is known that the management of such pathological conditions benefits both from the irrigation of the nasal cavities with isotonic saline solution and from medication with hypertonics.

Isotonic, or physiological, solution is defined as such because the percentage of sodium chloride present is 0.9%, (i.e., about 9 grams of sodium chloride for each liter of purified/demineralized water), the same concentration normally present in cells and in the physiological fluids of our body. For this reason it is also normally used, as well as for nasal washes or eye cleansing, to dilute the drugs used in therapies to be performed by aerosol or, in the hospital setting, in intravenous therapies (rehydration, drug delivery).

The hypertonic solution instead has a salt concentration higher than 0.9% (around 2.2-2.4%), therefore higher than our cells. This solution is generally recommended by doctors for nasal washes or irrigations in the event of colds in children and adults: the hypertonic solution will ensure that, by osmosis, the cells will release water which will favor the expulsion of the mucus accumulated inside the nasal mucosa. In other words, when this more concentrated solution of salts comes into contact with the nasal mucosa, it draws the excess water in the nasal mucosa by osmotic process and dilutes the mucus, making it more fluid.

As part of his research and development activity, the Applicant has experimentally shown that a different therapeutic efficacy can be obtained by resorting to a saline solution which instead of demineralized water makes use of a salt-bromine-iodine water, which is abundant in numerous spas in Italy.

Of marine origin, the salt-bromine-iodine waters mainly consist of sodium chloride, iodine and bromine, fundamental elements for the functioning of the body. At the level of the mucosa, they carry out three therapeutic actions:
- Vasodilation: with increased secretion of mucus and the serous component thereof.
- Eu-trophism of the mucosa: with increased local reactivity.
- Improved mucociliary clearance: first immunological barrier of the airways.

Therefore, decreasing the viscosity of the secretions, the salt-bromine-iodine water makes them more fluid, favoring the restoration of ciliary motility. Furthermore, being buffered hypertonic waters, they have the role of inhibiting, in the respiratory mucosa of the upper airways, the adhesion and replication of the main respiratory viruses (Rhinovirus, Adenovirus, Syncytial Respiratory Virus, Coronavirus), hindering the adhesion of the main pathogenic bacteria and the formation of biofilms, favoring the restoration of the normal microbiome of the nasopharynx.

These features make the salt-bromine-iodine waters, especially for children and the elderly, a natural therapeutic aid for the treatment and prevention of respiratory diseases of the upper airways.

Following the tests carried out, two formulas of salt-bromine-iodine thermal waters were developed, the first, an isotonic solution for nasal irrigations, to be used in combination with a nasal insufflator capable of performing a complete washing of both nasal cavities, the second, a hypertonic solution to be used with a nasal nebulizer capable of producing particles ranging in size between 70 and 10 microns, where the cumulative percentage of mass greater than 10 microns is over 98%. This fraction represents the amount of product which is not inhaled and which is therefore used only for the treatment of the upper airways without reaching the alveoli.

Therefore, the invention relates to a kit of two medical devices respectively consisting of:
A) an isotonic solution based on a salt-bromine-iodine thermal water, and a syringe with a multi-hole nasal nozzle specific for irrigating throughout the rhino-sinus-pharyngeal area; and
B) a hypertonic solution 2.5% based on a salt-bromine-iodine thermal water; and a syringe with a single-hole nasal nozzle specific for the nebulization of medicines in solution, capable of producing particles of a size between 70 and 10 microns, adapted to spraying with said hypertonic solution of the entire rhino-sinus-pharyngeal area, minimizing the risk of reaching the lower respiratory tract (lung penetration).

Further features and advantages of the invention will become apparent from the following description and experimental tests, with reference to the accompanying drawings which show, merely by way of non-limiting example, a preferred embodiment of the invention and graphs related to various tests carried out on the diameter measurement of the average volume of the aerosol with thermal water and diagrams illustrating the efficacy on the treatment of the upper airways of the therapy with two medical devices based on isotonic and hypertonic solution of salt-bromine-iodine thermal water, compared with therapy based on an isotonic and hypertonic saline solution in demineralized water.

In the drawings:

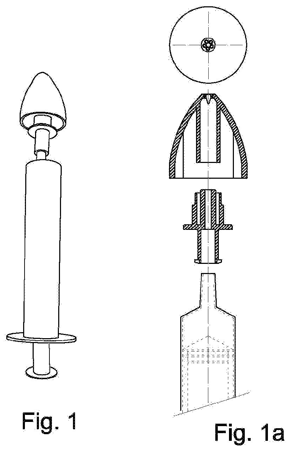

fig. 1 is an overall view of a syringe with a nasal nozzle specific for irrigating throughout the rhino-sinus-pharyngeal area;
fig. 1a shows diagrammatically sectional and plan views of the component elements of the syringe of fig. 1;

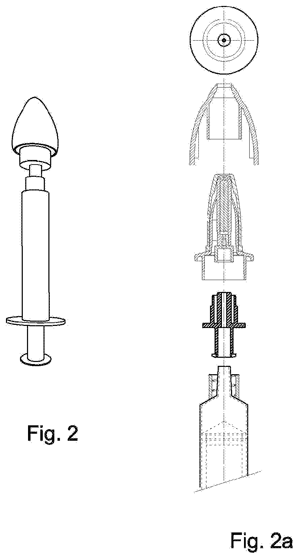

fig. 2 is an overall view of a syringe with a nasal nozzle specific for the nebulization of medicines in solution, adapted to medication throughout the rhino-sinus-pharyngeal area;
fig. 2a shows diagrammatically sectional and plan views of the component elements of the syringe of fig. 2;
figures 3, 4 and 5 show the graphs related to three tests on the particle distribution of the aerosol insufflator by means of laser diffraction (Low angle Laser Scattering, LALLS) of thermal water.
fig. 6 shows the diagram related to the efficacy of the therapy in terms of the frequency of colds;
fig. 7 shows the diagram related to the efficacy of the therapy in terms of frequency of allergic rhinitis;
fig. 8 shows the diagram related to the efficacy of the therapy in terms of stuffed nose;
fig. 9 shows the diagram related to the efficacy of the therapy in terms of runny nose;
fig. 10 shows the diagram related to the efficacy of the therapy in terms of the sensation of weight on the paranasal sinuses;
fig. 11 shows the diagram related to the efficacy of the therapy in terms of cough;
fig. 12 shows the diagram related to the efficacy of the therapy in terms of itching and tingling in the nose;
fig. 13 shows the diagram related to the efficacy of the therapy in terms of difficulty breathing;
fig. 14 shows a diagram related to the comparison between the efficacy of the therapy with thermal water and saline water in terms of the frequency of colds and allergic rhinitis;
fig. 15 shows a diagram related to the comparison between the efficacy of the therapy with thermal water and saline water in terms of stuffed nose, runny nose and sensation of pressure on the nasal sinuses;
fig. 16 shows a diagram related to the comparison between the efficacy of the therapy with thermal water and saline water in terms of cough, itching and tingling in the nose and difficulty breathing.

### DETAILED DESCRIPTION

The object of the present invention is a kit according to claim 1, comprising two isotonic and hypertonic salt-bromine-iodine thermal water formulas to be used with a medical device thereof so as to be able to perform, in separate modes, a nasal washing irrigation and a medicine nebulization.

Two distinct medical devices are therefore described below, characterized as follows:
FOR AN ISOTONIC SOLUTION:
   A medical device consisting of:
      a. an isotonic solution based on a salt-bromine-iodine thermal water;
      b. a syringe with multi-hole nasal nozzle specific for irrigating throughout the rhino-sinus-pharyngeal area.
   *Nasal irrigation* is indicated both for daily hygiene, favoring, with the hydration, mucociliary clearance, and for the mechanical removal of pathological mucus, a preparatory action required for the subsequent nasal medication, with the nebulizing device.
   The conical shape of the nozzle, made of soft and non-porous material, allows a perfect sealing to the nasal vestibule, ergonomically adapting to the nostrils of both children and adults.
FOR THE HYPERTONIC SOLUTION:
   A medical device consisting of:
   a. a hypertonic solution 2.5% based on a salt-bromine-iodine thermal water;
   b. a syringe with a single-hole nasal nozzle, specific for the nebulization of medicines in solution, capable of producing particles of a size 70-10 microns, adapted to spraying with said hypertonic solution of the whole rhino-sinus-pharyngeal area. The conical shape of the nozzle, made of soft and non-porous material, allows a perfect sealing to the nasal vestibule, ergonomically adapting to the nostrils of both children and adults.

### Evaluation of the median diameter of the mass

The object of the work was to verify the median diameter of the mass of an aerosol produced by a single-hole nasal nebulizer starting from a hypertonic thermal solution.

The Scattering method of low angle laser light was used, which is based on the fact that the diffraction angle is inversely proportional to the particle size. Such an analysis allows the determination of the size distribution of the aerosol particles directly by spraying the cloud through the laser beam.

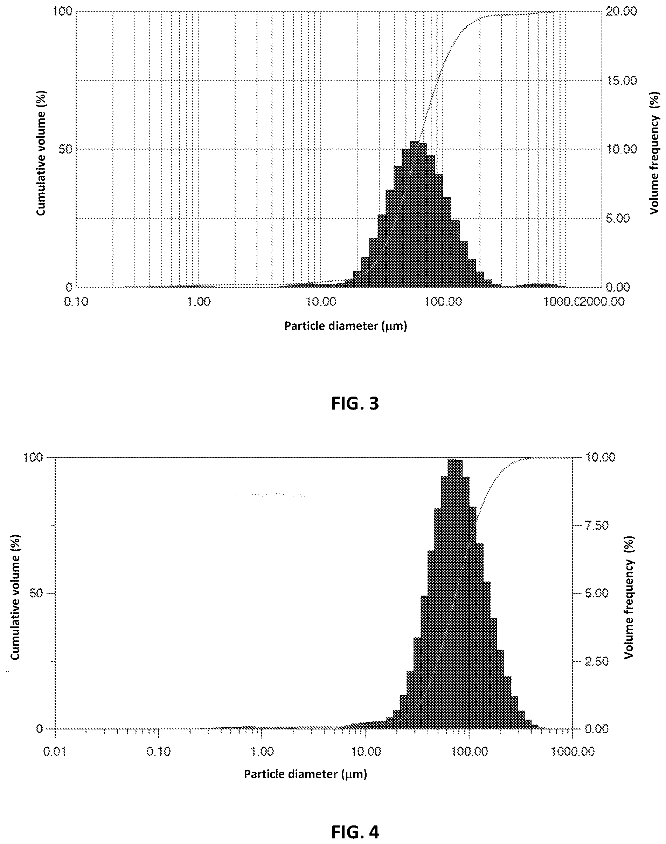

Three tests were carried out: the data are shown in the graphs of figures 3-5 and in the following table A, which shows the individual data of the aerosol produced with a salt-bromine-iodine thermal water solution. The diameter values are in µm. The value of the distribution spread (Span) and the "Residual" are also indicated, i.e., the quality of the mathematical model used (less than 1%).

**TABLE A**

| **Test** | **D _{[v,0.1]} (µm)** | **D _{[v,0.5]} (µm)** | **D _{[v,0.9]} (µm)** | **Residual (%)** | **Span** |
|---|---|---|---|---|---|
| Test 1 | 29.1 | 61.4 | 129.5 | 0,25 | 1.64 |
| Test 2 | 33.9 | 74.7 | 166.2 | 0,25 | 1.77 |
| Test 3 | 31.1 | 68.9 | 163.0 | 0.19 | 1.91 |
| **Average ± Standard dev.** | **31.1 ± 2.4** | **68.9 ± 6.7** | **163.0 ± 20.3** | | |

The results table shows the median diameter of volume D_{[v,0.5]} (MMD), the percentages of undersized particles D_{[V,0,1]}, while D _{[v, 0.9]} indicates the values of the diameters which correspond respectively to 10% and 90% of the particle population.

As can be seen in table A, which compares the data of the three tests carried out, the dimensional distribution MMD (in microns) of the aerosol particles produced by the nebulizer used with the hypertonic solution based on salt-bromine-iodine thermal water, is equal to 68.9 µm while D_{[,0.9]} is about 160 µm. The aerosol size profile has a homogeneous distribution for all size ranges during the nebulization of the entire volume of liquid and proves to be optimal for nasal deposition, preventing particles from penetrating the lung.

In conclusion, the aerosol of hypertonic solution in salt-bromine-iodine thermal water emitted by the nebulizer has a cumulative percentage of mass greater than 10 microns over 98%. This fraction represents the amount of product not inhaled and specifically useful for the treatment of the upper airways.

### CURRENT THERAPIES FOR UPPER AIRWAY DISEASES

The burden and cost of inflammatory, infectious and allergic diseases involving the upper airways are increasing worldwide. Therefore, strategies must change management to counter the transformation of the healthcare system for integrated care (19). In this regard, integrated care pathways are multi-disciplinary care plans (20) and promote the application of guidelines recommendations for clinical practice (21,22), with a multidisciplinary approach, which should include both diagnostic procedures and therapeutic strategies.

There is evidence that several therapeutic options may be effective in treating upper airway diseases, as demonstrated by numerous studies which have investigated the efficacy and safety of different drug classes. In this regard, a recent summary review of a series of studies, conducted in Italy and in the real world, exploring the innovative use of conventional drugs or the clinical application of complementary medicine, was published (January 2020) in the official journal of the Italian Society of Pediatrics.

Among the drugs studied, hypertonic saline solutions and thermal waters play a very important role.

In fact, while nasal irrigation with hypertonic saline solution significantly reduced the size of the adenoid in children with adenoid hypertrophy (23), salt-sulfur thermal water has been used successfully in children with recurrent respiratory infections (24) and salt-bromine-iodine thermal water reduced cough from post-nasal drip in children with acute upper airway infection (25).

In the article "Behavior of Serum IgE and IgA in Patients with Allergic Rhinitis Treated with Iodine Bromide Thermal Water", Barbieri M et al, on ACTA OTORHINO LARYNGOLOGICA ITALICA, PACINI EDITORE, PISA, volume 22 no.4, August 2002, the anti-inflammatory effect is described of a hypertonic solution comprising salt-bromine-iodine (SBI) thermal water administered via intranasal spray, by means of a generic dispenser in the longitudinal axis coaxial to the direction of the nasal cavity.

The article "Efficacy of inhalation therapy with water of Salsomaggiore (Italy) in chronic and recurrent nasosinusal inflammation treatment", Passali F M et al on LA CLINICA TERAPEUTICA, ITALY, vol. 159, no. 3, May 2008, describes a hypertonic solution comprising SBI thermal water, administered via nebulization.

### EXPERIMENTAL ASSESSMENT

The objective of the study underlying the present invention was to highlight the efficacy of a therapy with:
- Two medical devices based on isotonic and hypertonic solution of salt-bromine-iodine thermal water performed for 15 days, the first with a nasal nozzle provided with five holes with a diameter of 0.50-0.55 mm and the second with a single-hole nasal nozzle with a diameter of about 2 mm, on the treatment of inflammation of the upper airways.
- two medical devices identical to the previous ones, based on isotonic and hypertonic saline solution performed for 15 days, on the treatment of inflammation of the upper airways.

### Primary endpoint

Verification of efficacy in terms of:
- Clinical assessment of the predominant symptoms (score from 0 to 10 in terms of increasing intensity):
   - Stuffed nose
   - Runny nose
   - Sensation of pressure on the paranasal sinuses
   - Itchy and tingling nose
   - Cough
   - Difficulty breathing

### Secondary endpoint

- Presence of colds and/or allergic rhinitis
- Compare the effectiveness of isotonic and hypertonic thermal water based devices with isotonic and hypertonic saline water based devices.

### DURATION OF THE ASSESSMENT:

The patients enrolled in the study were divided into 5 groups.
a. The first group treated with an administration for two weeks, with a single vial of isotonic salt-bromine-iodine thermal water solution to irrigate twice a day.
b. The second group treated with an administration for two weeks with the use of the device and isotonic salt-bromine-iodine thermal water solution to irrigate twice a day.
c. The third group treated with an administration for two weeks with the use of the device and isotonic solution to irrigate twice a day.
d. The fourth group treated with an administration for two weeks with the use of the device and isotonic salt-bromine-iodine water solution for irrigation and the use of the device and hypertonic salt-bromine-iodine thermal water solution for nebulization twice a day.
e. The fifth group treated with an administration for two weeks with the use of the device and isotonic saline solution for irrigation and the use of the device and hypertonic saline solution for nebulization twice a day.

### MATERIALS AND METHODS:

### Inclusion criteria:

Patients aged 3 to 12 years and older with inflammation of the three upper airway areas were recruited.

### Exclusion criteria:

All patients with suspected arterial hypertension, hemodynamically unstable or with hemoglobin less than 10 g/dl were excluded.

10 ml vials (strips) of isotonic solution of salt-bromine-iodine thermal water were used.

Four medical devices were used:
- One based on isotonic solution of salt-bromine-iodine thermal water.
- One based on isotonic saline solution.
- One based on hypertonic solution of salt-bromine-iodine thermal water.
- One based on hypertonic saline solution.

Number of patients recruited for the first group: 20 patients.

Number of patients recruited for the second group: 20 patients.

Number of patients recruited for the third group: 20 patients.

Number of patients recruited for the fourth group: 20 patients.

Number of patients recruited for the fifth group: 20 patients.

All the patients first underwent an initial enrollment visit to collect personal data on psychosocial factors, occupational status, family and medical history, lifestyle, medical records, and health status.

All patients were asked to fill in a diary containing the following form:
Stuffed nose
Runny nose
Sensation of pressure on the nasal sinuses
Itchy and tingling nose
Cough
Difficulty breathing

### Complications

Particular attention was paid to the profile of side effects encountered during treatment. In this regard, no clinically significant phenomenon was found which was associated with the administration of medical devices in order to confirm the extreme tolerability of the components and formulation.

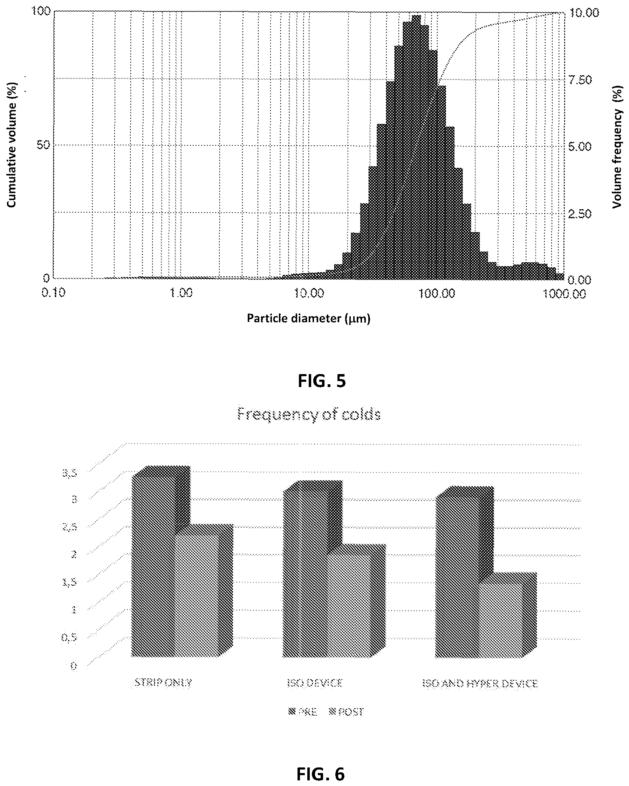

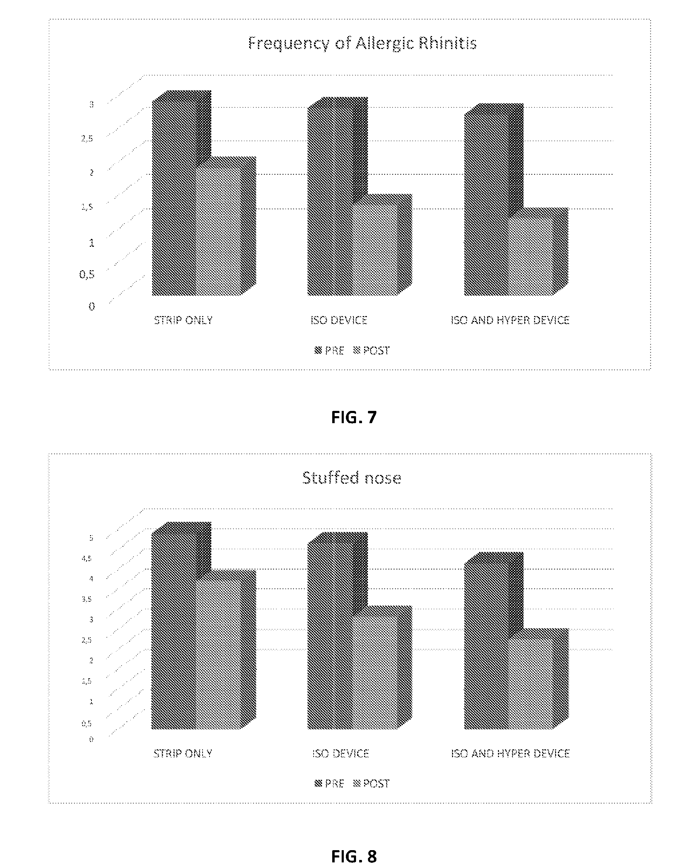

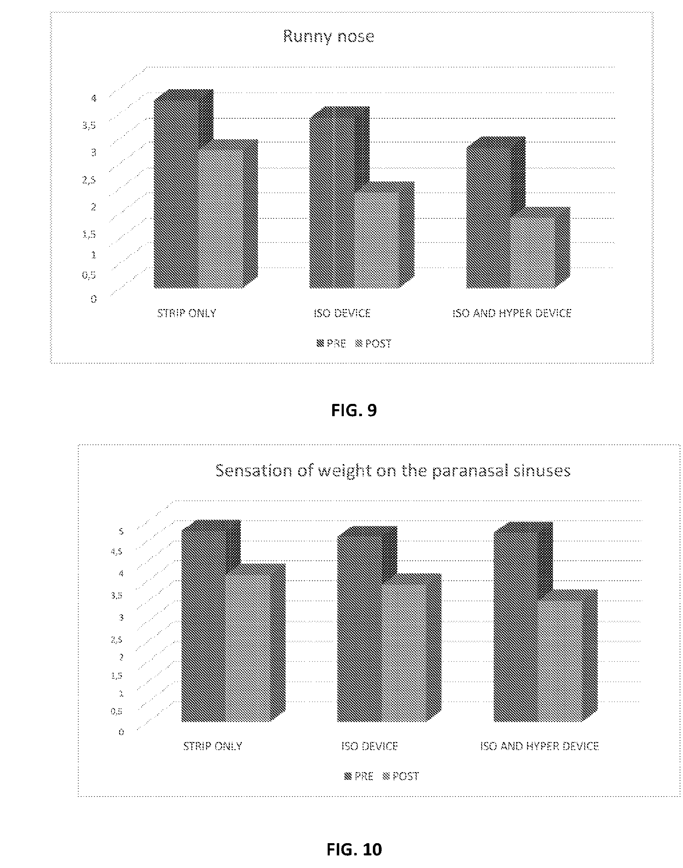

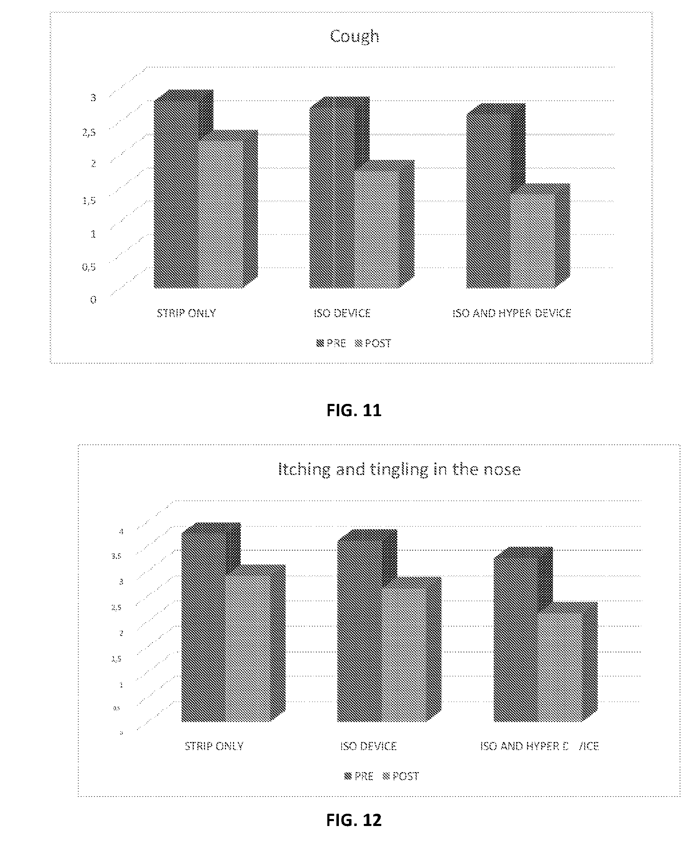

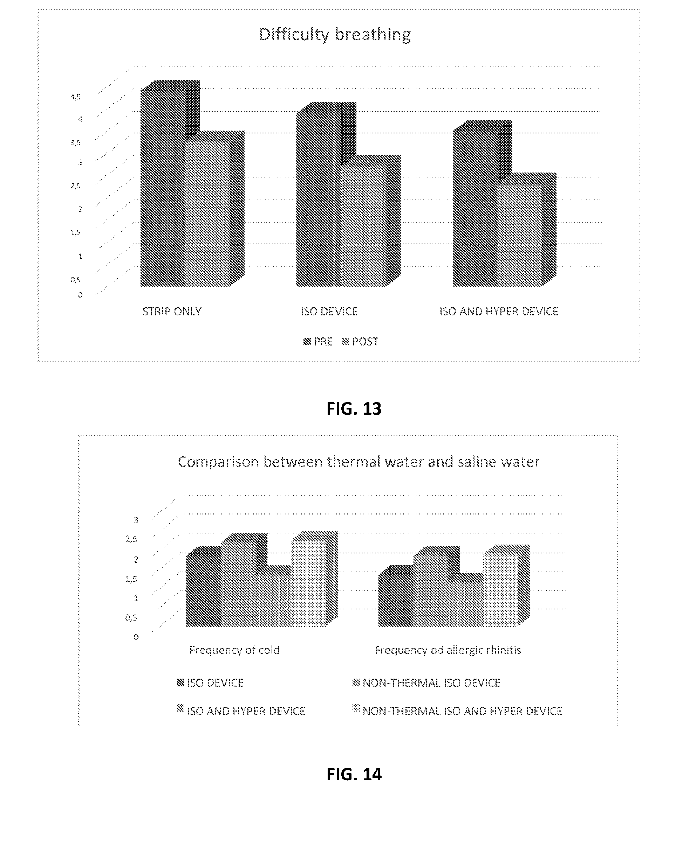

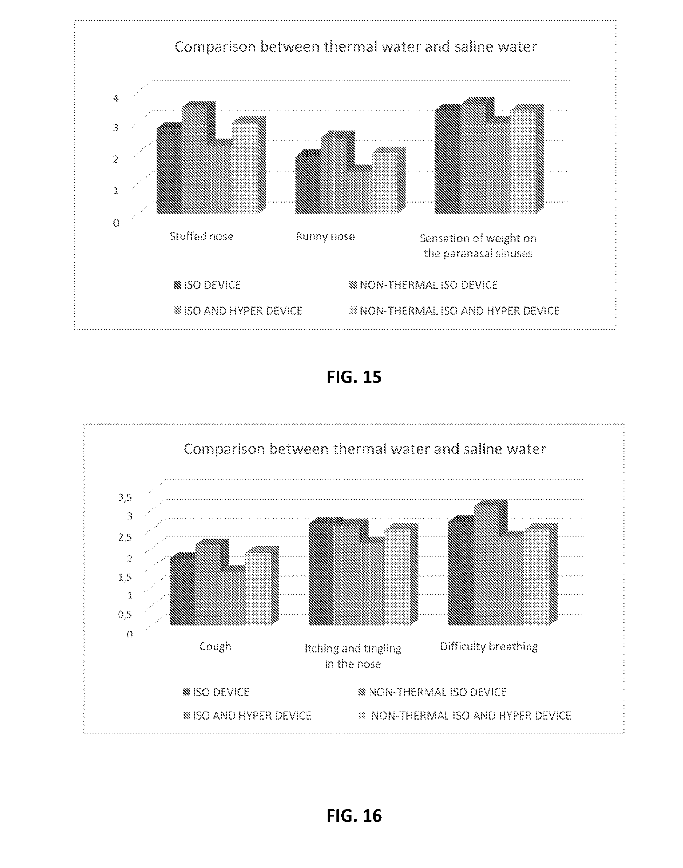

The results are reported in the diagrams of figures 6-16 which also compare the use of thermal water with respect to physiological saline water.

Such data are compared in table B below, which shows the improvement in symptoms as a percentage.

**TABLE B**

| **% IMPROVEMENT OF SYMPTOMS** | | | | | |
|---|---|---|---|---|---|
| | **STRIP ONLY** | **ISO DEVICE** | **ISO DEVICE NO THERMAL** | **ISO AND HYPER DEVICE** | **DEVICE ISO AND HYPER NO THERMAL** |
| **Cold frequency** | 32.3 | 38.3 | 31.2 | 53.4 | 31.8 |
| **Frequency of allergic rhinitis** | 34.5 | 51.8 | 40.3 | 57.4 | 38.7 |
| **Stuffed nose** | 24.5 | 39.8 | 27.8 | 45.8 | 30.2 |
| **Runny nose** | 26.6 | 44.1 | 30.5 | 50 | 37.5 |
| **Sensation of pressure on the nasal sinuses** | 23.1 | 26.1 | 22.6 | 36.2 | 29.2 |
| **Cough** | 21.4 | 35.2 | 30 | 46.1 | 36.6 |
| **Itchy and tingling nose** | 22.6 | 26.4 | 23.5 | 33.8 | 28.6 |
| **Difficulty breathing** | 26.1 | 30.8 | 26.2 | 34.3 | 32.4 |

### Conclusion

Inflammatory, allergic or infectious diseases of the upper airways must be treated with effective and safe drugs. Respiratory infections deserve proper observation, antibiotics should be prescribed carefully, and the preventive strategy should be preferred. Allergic disorders can be successfully treated with specific therapy and allergic symptoms can be optimally relieved with medication.

Considering that inflammation, infection and allergy often coexist, a multi-disciplinary approach integrated into clinical practice should always be implemented, in which conventional treatment can be appropriately associated and integrated with complementary medicine.

Among the complementary drugs, the tests carried out have shown the possibility of using thermal waters to be of great interest, both for irrigation and for the treatment of the rhino-sinus-pharyngeal area, interfering in the three fundamental pathogenetic aspects: blocking, inflammation and infection.

In fact, by irrigating the nose with the isotonic solution and, subsequently, medicating it with the hypertonic solution, the unblocking, anti-inflammation and disinfection of the nasal area will be obtained, on the *health* of which the "well-being" of the entire respiratory system depends.

From the data collected, it is apparent that the best therapeutic performance is obtained with hypertonic solutions with respect to isotonic ones.

In addition to having the requisites of NaCl hypertonics (decongestion by osmosis, increased phagocyte activity, increased secretory IgA), being iodized, thermal hypertonics have a remarkable antiviral power (reducing both intracellular penetration and endocellular replication), in addition to bacterial anti-adhesion action. Furthermore, recent scientific evidence has highlighted the ability to "normalize" the nasopharyngeal microbiome, improving the recurrence of respiratory diseases. Not to mention the natural buffering action thereof due to the pH thereof tending to basic. If with the irrigation effect the mechanical removal of pathological secretions and the restoration of mucociliary clearance are obtained, nebulization, with a specific device for the nasal area, particularly suitable for the nasopharynx, is the real physio-pathological carrefour.

From the comparison of the use of the device based on isotonic and hypertonic thermal water and of the device based on isotonic and hypertonic saline water to allow the medication of the rhinogenic cause of respiratory diseases, associating the specific device with a buffered hypertonic thermal water, assumes great significance in the field of upper airway therapy, both curative and preventive.

### Bibliography

1) Pitt E, Gallegos D, Comans T, Cameron C, Thornton L. Exploring the influence of local food environments on food behaviours: a systematic review of qualitative literature. Public Health Nutr. 2017;20:2393-405
2) Brennan A, Akehurst R. Modelling in health economic evaluation. What is its place? What is its value? Pharmacoeconomics. 2000;17:445-59
3) Use of real-world evidence to support regulatory decision-making for medical devices. Guidance for industry and Food and Drug Administration staff document issued on August 31, 2017. Bethesda: US Food and Drug Administration, US Department of Health and Human Services Food and Drug Administration, Center for Devices and Radiological Health Center for Biologics Evaluation and Research; 2017
4) Sherman RE, Anderson SA, Dal Pan GJ, Gray GW, Gross T, Hunter NL, et al. Real-world evidence-what is it and what can it tell us? N Engl J Med 2016; 375:2293-7
5) Ciprandi G, Cirillo I. The lower airway pathology of rhinitis. J Allergy Clin Immunol 2006;118:1105-9
6) Ciprandi G, Caimmi D, Miraglia del Giudice M, La Rosa M, Salpietro C, Marseglia G, et al. Recent developments in United airways disease. Allergy Asthma Immunol Res 2012;4:171-7
7) Fahey T, Stocks M, Thomas T. Systematic review of the treatment of upper respiratory tract infection. Arch Dis Child 1998;79:225-30
8) Wald ER, Guerra N, Byers C. Upper respiratory tract infections in young children: duration and frequency of complications. Pediatrics 1991;87:129
9) Wigand ME. Endonasal sinus surgery with endoscopical control: from radical operation to rehabilitation of the mucosa. Endoscopy 1978;10:255-60
10) Dykewicz MS. Rhinitis and sinusitis. J Allergy Clin Immunol 2003;111:S520-9
11) Watkins DS, Lewis RH, Bascalin KA, et al. Expression and localization of the inducible isoform of nitric oxide synthase in nasal epithelium. Clin Exp Allergy 1998;28:211-9
12) Djiupesland PG, Chatkin JM, Qian W, et al. Nitric oxide in nasal airway: a new dimension in otorhinolaryngology. Am J Otolaryngol 2001;22:19-32
13) Brandzaeg P. Immunobarriers of the mucosa of the upper respiratory and digestive pathways. Acta Otolaryngol (Stockh) 1988;105:172-80
14) Stankiewicz J. Chronic sinusitis. In Johnson JT, Yu VL eds. Infectious diseases and antimicrobial therapy of the ears, nose and throat. WB Saunders Co. Philadelphia, 1997
15) Ciprandi G, Buscaglia S, Pesce G. Minimal persistent inflammation is present at mucosal level in patients with asymptomatic rhinitis and mite allergy. J Allergy Clin Immunol 1995;96:971-9
16) Randall D. Wolcott; Garth D. Ehrlich. Biofilms and Chronic Infections. JAMA. 2008;299:2682-4
17) Zuliani G, Carron M, Gurrola J, Coleman C, Haupert M, Berk R, et al. Identification of adenoid biofilms in chronic rhinosinusitis. Int J Pediatr Otorhinolaryngol 2006;70:1613-7.
18) Ivarsson M, Ebenfelt A, Lundberg C. Do the leukocytes in the surface secretion on the adenoid have an immunological function?. Acta Otolaryngol (Stockh)1997;117:872-8
19) Bousquet J, Pfaar O, Togias A, Schunemann HJ, Ansotegui I, Papadopoulos NG, et al. 2019 ARIA Care pathways for allergen immunotherapy. Allery 2019 (in press)
20) Campbell H, Hotchkiss R, Bradshaw N, Porteous M. Integrated care pathways. BMJ. 1998;316(7125) :133-137
21) Hujala A, Taskinen H. How to support integration to promote care for people with multimorbidity in Europe? In: Rissanen S, Richardson E, vanGinneken E, eds. European Observatory Policy Briefs. Copenhagen;2017. http://www.euro.who.int/_data/asset s/pdf_file/0008/337589/PB_26.pdf. Accessed November 2019
22) Palmer K, Marengoni A, Forjaz MJ, et al. Multimorbidity care model: Recommendations from the consensus meeting of the Joint Action on Chronic Diseases and Promoting Healthy Ageing across the Life Cycle (JA-CHRODIS). Health Policy. 2018; 122 (1) :4-11
23) Ciprandi G, Varricchio A, Capasso M, Varricchio AM, De Lucia A, Ascione E, et al. Hypertonic saline solution in children with Adenoidal Hypertrophy: preliminary evidence. Eur J Inflamm 2007;5:159-63.
24) Varricchio A, Giuliano M, Capasso M, Del Gaizo D, Ascione E, De Lucia A, et al. Salso-sulphide thermal water in the prevention of Recurrent Respiratory Infections in children. Int J Immunopathol Pharmacol 2013;26:941-52 .
25) La Mantia I, Ciprandi G, Varricchio A, Cupido F, Andaloro C. Salso-bromo-iodine thermal water: a nonpharmacological alternative treatment for postnasal drip-related cough in children with upper respiratory tract infections. J Biol Reg 2018; 32(1 Suppl. 2):41-47.
26) La Mantia I. & Andaloro C. (2018). Effects of salso-bromo-iodine thermal water in children suffering from otitis media with effusion: a randomized controlled pilot study. La Clinica Terapeutica, 169(1), e10-e13.
27) Malizia V1, Fasola S, Ferrante G, Cilluffo G, Montalbano L, Landi M, Marchese D, Passalacqua G, La Grutta S. Efficacy of Buffered Hypertonic Saline Nasal Irrigation for Nasal Symptoms in Children with Seasonal Allergic Rhinitis: A Randomized Controlled Trial. Int Arch Allergy Immunol. 2017;174(2):97-103.
28) Pellegrini M., Fanin D., Nowicki Y., Guarnieri G., Bordin A., Faggian D., Plebani M., Saetta M. & Maestrelli P. (2005). Effect of inhalation of thermal water on airway inflammation in chronic obstructive pulmonary disease. Respiratory medicine, 99(6), 748-754.
29) Keller S., König V. & Mösges R. (2014). Thermal water applications in the treatment of upper respiratory tract diseases: a systematic review and meta-analysis. J Allergy (Cairo). 2014:943824.
30) Passali D., Lauriello M., Passali G. C., Passali F. M., Cassano M., Cassano P. & Bellussi L. (2008). Clinical evaluation of the efficacy of Salsomaggiore (Italy) thermal water in the treatment of rhinosinusal pathologies.
31) Passali F. M., Crisanti A., Passali G., Cianfrone F., Bocchi M., Messineo G., Bellussi L. & Passali D. (2008). Efficacy of inhalation therapy with water of Salsomaggiore (Italy) in chronic and recurrent nasosinusal inflammation treatment.
32) Cupido G.F., Gelardi M., La Mantia I., Aragona S.E., Vicini C. & Ciprandi G. (2019). Otorhinolaryngological Disorders ISGOI. Broncalt®, class II medical device, in patients with acute upper airways disease: a survey in clinical practice. Acta Biomed. 90(7-S):24-29.
33) Mora R., Salami A., Casazza A., Passali G. C., Cordone M. P., Mora M. F. & Barbieri M. (2003). Treatment of Specific Allergic Rhinitis with Salso-bromo-iodine Water: One Year of Therapy. @ rq. otorrinolaringol, 7(3), 220-223.
34) GeLardi G., Carpentieri A., Cristiano F. & Lampa E. (2014). Crenoterapia con acqua. Salso-Solfato-Bicarbonato-Al-calina sorgiva in Rapolla (PZ) nelle patologie delle alte e basse vie respiratorie: studio clinico-sperimentale. Clin. Term, 61(1-2), 6-14.
35) Šlapak I., Skoupá J., Strnad P. & Hornik P. (2008). Efficacy of isotonic nasal wash (seawater) in the treatment and prevention of rhinitis in children. Archives of otolaryngology-head & neck surgery, 134(1), 67-74.
36) Pollastrini L. & Carluccio F. (1994). Gli effetti delle acque solfureosalsobromoiodiche sulle flogosi catarrali delle vie aeree superiori. Acta Otorhinolaringologica italica, (suppl 44).
37) Fenu G., Bozzo C., Carai A., Delehaye E., Foddai M., Meloni F. & Montella A. C. (2010). Effects of isotonic salso-bromo-iodine thermal water after sinunasal surgery: a preliminary morphological study. The journal of alternative and complementary medicine, 16(4), 341-343.
38) Staffieri A., Miani C., Bergamin A. M., Arcangeli P. & Canzi P. (1998). Effect of sulfur salt-bromine-iodine thermal waters on albumin and IgA concentrations in nasal secretions. Acta otorhinolaryngologica Italica: organo ufficiale della Societa italiana di otorinolaringologia e chirurgia cervico-facciale, 18(4), 233-238.
39) Ottaviano G., Marioni G., Staffieri C., Giacomelli L., Marchese-Ragona R., Bertolin A. & Staffieri A. (2011). Effects of sulfurous, salty, bromic, iodic thermal water nasal irrigations in nonallergic chronic rhinosinusitis: a prospective, randomized, double-blind, clinical, and cytological study. American journal of otolaryngology, 32(3), 235-239.
40) Passariello A., Di Costanzo M., Terrin G., Iannotti A., Buono P., Balestrieri U., Balestrieri G., Ascione E., Pedata M., Canani F. B. & Canani R. B. (2012). Crenotherapy modulates the expression of proinflammatory cytokines and immunoregulatory peptides in nasal secretions of children with chronic rhinosinusitis. American journal of rhinology & allergy, 26(1), e15-e19.
41) Staffieri A. & Abramo A. (2007). Sulphurous-arsenical-ferruginous (thermal) water inhalations reduce nasal respiratory.

## Claims

1. A kit of two medical devices A) and B) respectively consisting of:
A) an isotonic solution based on a salt-bromine-iodine thermal water, and
a syringe with a multi-hole nasal nozzle specific for irrigating said solution throughout the rhino-sinus-pharyngeal area;
and of:
B) a hypertonic solution 2.5% based on a salt-bromine-iodine thermal water; and
a syringe with a single-hole nasal nozzle specific for the nebulization of medicines in solution, capable of producing particles of a size between 70 and 10 microns, adapted to spraying with said hypertonic solution of the entire rhino-sinus-pharyngeal area, minimizing the risk of reaching the lower respiratory tract.

2. A kit of two medical devices according to claim 1, wherein the first device A) for nasal irrigation favors, with the hydration, both mucociliary clearance and mechanical removal of pathological mucus, a preparatory action required for the subsequent nasal spraying, with the nebulizing device B).

3. A kit of two medical devices according to claim 1, **characterized in that** the syringe of the first device A) has a conical nasal nozzle made of soft, non-porous material which allows the perfect sealing to the nasal vestibule, ergonomically adapting to the nostrils of both children and adults, said nasal nozzle being provided at the beveled tip thereof with five coplanar holes arranged in a crown about the axis of said ogival nozzle capable of splitting the outflowing jet, the diameter of each hole being between 0.45 and 0.55 mm.

4. A kit of two medical devices according to claim 1, **characterized in that** the syringe of the second device B) has a conical nasal nozzle made of soft, non-porous material which allows the perfect sealing to the nasal vestibule, ergonomically adapting to the nostrils of both children and adults, said nasal nozzle being provided at the beveled tip thereof with a single hole of about 1.8-2.2 mm in diameter.

5. A kit of two medical devices according to claim 1, **characterized in that** the nebulization of a buffered hypertonic solution through the second medical device B), in addition to having the requisites of Nacl hypertonics, has a high antiviral power by reducing both intracellular penetration and endocellular replication in addition to providing a bacterial anti-adhesion action.

6. A kit of two medical devices according to claim 1, for use in a treatment method for inflammation of the rhino-sinus-pharyngeal area.

7. An association of an isotonic solution based on a salt-bromine-iodine thermal water, and a hypertonic solution 2.5% based on a salt-bromine-iodine thermal water for use in a treatment method in two phases of the inflammation of the rhino-sinus-pharyngeal area , first by irrigating the nose with an isotonic solution by means of a syringe with a multi-hole nasal nozzle specific for irrigating said solution throughout the rhino-sinus-pharyngeal area, and subsequently, spraying it with a hypertonic solution, by means of a syringe with a single-hole nasal nozzle specific for the nebulization of medicines in solution, capable of producing particles of a size between 70 and 10 microns, adapted to the spraying with said hypertonic solution of the entire rhino-sinus-pharyngeal area, in order to obtain unblocking, anti-inflammation and disinfection of the nasal area, on the health of which the "well-being" of the entire respiratory system depends, minimizing the risk of reaching the lower respiratory tract.

## Patentansprüche

1. Satz von zwei medizinischen Geräten A) und B), die jeweils aus Folgendem bestehen:
A) einer isotonischen Lösung auf der Grundlage eines Salz-Brom-Iod-Thermalwassers und
einer Spritze mit einer Mehrloch-Nasendüse, spezifisch zum Spülen der Lösung durch den gesamten Nasen-Nebenhöhlen-Rachen-Bereich,
und aus:
B) einer 2,5-prozentigen hypertonischen Lösung auf der Grundlage eines Salz-Brom-Iod-Thermalwassers und,
einer Spritze mit einer Einzelloch-Nasendüse, spezifisch für die Vernebelung von Medikamenten in Lösung, die dazu in der Lage ist, Teilchen mit einer Größe zwischen 70 und 10 Mikrometer zu erzeugen, angepasst an das Sprühen des gesamten Nasen-Nebenhöhlen-Rachen-Bereichs mit der hypertonischen Lösung, wobei die Gefahr minimiert wird, die unteren Atemwege zu erreichen.

2. Satz von zwei medizinischen Geräten nach Anspruch 1, **dadurch gekennzeichnet, dass** das erste Gerät A) zur Nasenspülung, mit der Hydratation, sowohl die mukoziliäre Reinigung als auch die mechanische Entfernung von pathologischem Schleim begünstigt, eine Vorbereitungshandlung, die für das anschließende Nasensprühen, mit dem Vernebelungsgerät B), erforderlich ist.

3. Satz von zwei medizinischen Geräten nach Anspruch 1, **dadurch gekennzeichnet, dass** die Spritze des ersten Geräts A) eine konische, aus einem weichen, nicht-porösen Material hergestellte Nasendüse aufweist, welche die vollkommene Abdichtung gegenüber dem Naseneingang ermöglicht, wobei sie sich ergonomisch an die Nasenlöcher von sowohl Kindern als auch Erwachsenen anpasst, wobei die Nasendüse an der abgeschrägten Spitze derselben mit fünf komplanaren, in einem Kranz um die Achse der spitzovalen Düse angeordneten Löchern versehen ist, die in der Lage sind, den ausströmenden Strahl zu teilen, wobei der Durchmesser jedes Lochs zwischen 0,45 und 0,55 mm beträgt.

4. Satz von zwei medizinischen Geräten nach Anspruch 1, **dadurch gekennzeichnet, dass** die Spritze des zweiten medizinischen Geräts B) eine konische, aus einem weichen, nicht-porösen Material hergestellte Nasendüse aufweist, welche die vollkommene Abdichtung gegenüber dem Naseneingang ermöglicht, wobei sie sich ergonomisch an die Nasenlöcher von sowohl Kindern als auch Erwachsenen anpasst, wobei die Nasendüse an der abgeschrägten Spitze derselben mit einem einzelnen Loch von etwa 1,8 bis 2,2 mm im Durchmesser versehen ist.

5. Satz von zwei medizinischen Geräten nach Anspruch 1, **dadurch gekennzeichnet, dass** die Vernebelung einer gepufferten hypertonischen Lösung durch das zweite medizinische Gerät B), zusätzlich dazu, dass sie die Voraussetzungen von NaCl-Hypertonie aufweist, eine hohe antivirale Kraft aufweist, durch Verringern sowohl des intrazellulären Eindringens als auch der endozellulären Replikation, zusätzlich zum Bereitstellen einer bakteriellen Antiadhäsionswirkung.

6. Satz von zwei medizinischen Geräten nach Anspruch 1, zur Verwendung bei einem Behandlungsverfahren für eine Entzündung des Nasen-Nebenhöhlen-Rachenbereichs.

7. Verbindung einer isotonischen Lösung auf der Grundlage eines Salz-Brom-Iod-Thermalwassers und einer 2,5-prozentigen hypertonischen Lösung auf der Grundlage eines Salz-Brom-Iod-Thermalwassers zur Verwendung bei einem Behandlungsverfahren in zwei Phasen der Entzündung des Nasen-Nebenhöhlen-Rachenbereichs, zuerst durch Spülen der Nase mit einer isotonischen Lösung mit Hilfe einer Spritze mit einer Mehrloch-Nasendüse, spezifisch zum Spülen der Lösung durch den gesamten Nasen-Nebenhöhlen-Rachen-Bereich, und anschließend Sprühen derselben mit einer hypertonischen Lösung, mit Hilfe einer Spritze mit einer Einzelloch-Nasendüse, spezifisch für die Vernebelung von Medikamenten in Lösung, die dazu in der Lage ist, Teilchen mit einer Größe zwischen 70 und 10 Mikrometer zu erzeugen, angepasst an das Sprühen des gesamten Nasen-Nebenhöhlen-Rachen-Bereichs mit der hypertonischen Lösung, um Befreiung, Entzündungshemmung und Desinfektion des Nasenbereichs zu erreichen, von dessen Gesundheit das "Wohlbefinden" des gesamten Atmungssystems abhängt, wobei die Gefahr minimiert wird, die unteren Atemwege zu erreichen.

## Revendications

1. Kit de deux dispositifs médicaux A) et B) constitués respectivement de :
A) une solution isotonique à base d'une eau thermale de sel-brome-iode, et
une seringue munie d'une buse nasale multitrous spécifique pour irriguer toute la zone rhino-sinuso-pharyngée avec ladite solution ;
et de :
B) une solution hypertonique à 2,5 % à base d'une eau thermale de sel-brome-iode ; et
une seringue munie d'une buse nasale monotrou spécifique pour la nébulisation de médicaments en solution, capable de produire des particules d'une taille comprise entre 70 et 10 microns, adaptée à la pulvérisation avec ladite solution hypertonique de toute la zone rhino-sinuso-pharyngée, minimisant le risque d'atteindre les voies respiratoires inférieures.

2. Kit de deux dispositifs médicaux selon la revendication 1, dans lequel le premier dispositif A) d'irrigation nasale favorise, avec l'hydratation, à la fois la clairance mucociliaire et l'élimination mécanique de mucus pathologique, une action préparatoire requise pour la pulvérisation nasale ultérieure, avec le dispositif de nébulisation B).

3. Kit de deux dispositifs médicaux selon la revendication 1, **caractérisé en ce que** la seringue du premier dispositif A) présente une buse nasale conique en matériau souple, non poreux qui permet l'étanchéité parfaite au vestibule nasal, s'adaptant ergonomiquement aux narines tant des enfants que des adultes, ladite buse nasale étant dotée à son extrémité biseautée de cinq trous coplanaires disposés en couronne autour de l'axe de ladite buse ovale apte à séparer le jet sortant, le diamètre de chaque trou étant compris entre 0,45 et 0,55 mm.

4. Kit de deux dispositifs médicaux selon la revendication 1, **caractérisé en ce que** la seringue du second dispositif B) présente une buse nasale conique en matériau souple non poreux qui permet une étanchéité parfaite au vestibule nasal, s'adaptant ergonomiquement aux narines tant des enfants et que des adultes, ladite buse nasale étant dotée à son extrémité biseautée d'un seul trou d'environ 1,8 à 2,2 mm de diamètre.

5. Kit de deux dispositifs médicaux selon la revendication 1, **caractérisé en ce que** la nébulisation d'une solution hypertonique tamponnée à travers le second dispositif médical B), en plus de présenter les exigences d'hypertoniques de Nacl, présente un pouvoir antiviral élevé en réduisant à la fois la pénétration intracellulaire et la réplication endocellulaire en plus de fournir une action antiadhésive bactérienne.

6. Kit de deux dispositifs médicaux selon la revendication 1, pour une utilisation dans un procédé de traitement de l'inflammation de la zone rhino-sinuso-pharyngée.

7. Association d'une solution isotonique à base d'une eau thermale à base de sel-brome-iode, et d'une solution hypertonique à 2,5 % à base d'une eau thermale de sel-brome-iode pour une utilisation dans un procédé de traitement en deux phases de l'inflammation de la zone rhino-sinuso-pharyngée, d'abord en irriguant le nez avec une solution isotonique au moyen d'une seringue munie d'une buse nasale multitrous spécifique pour irriguer toute la zone rhino-sinuso-pharyngée avec ladite solution, puis en la vaporisant avec une solution hypertonique, au moyen d'une seringue munie d'une buse nasale monotrou spécifique pour la nébulisation de médicaments en solution, capable de produire des particules d'une taille comprise entre 70 et 10 microns, adaptée à la pulvérisation avec ladite solution hypertonique de toute la zone rhino-sinuso-pharyngée, afin d'obtenir le déblocage, l'anti-inflammation et la désinfection de la zone nasale, de la santé de laquelle dépend le « bien-être » de l'ensemble du système respiratoire, minimisant le risque d'atteindre les voies respiratoires inférieures.
